# EUROPEAN PATENT APPLICATION

(11) **EP 4 273 253 A1**
(43) Date of publication of application: **08.11.2023**
(21) Application number: 23167624.8
(22) Date of filing: 12.04.2023
(51) Int. Cl.: C12P 5/02, C12P 39/00, C12M 1/107, C12M 1/00

(54) **AN ANAEROBIC PROCESS FOR PRODUCTION OF METHANE RICH BIOGAS**

(30) Priority: 06.05.2022 IN 202221026442
(71) Applicant: Indian Oil Corporation Limited, 400 051 Mumbai Bandra (East) (IN)
(72) Inventor: KUMAR, Manoj, 121007 Faridabad (IN); SANDIPAM, Srikanth, 121007 Faridabad (IN); SINGH, Dheer, 121007 Faridabad (IN); GUPTA, Ravi Prakash, 121007 Faridabad (IN); BHATTACHARYYA, Debasis, 121007 Faridabad (IN); RAMAKUMAR, Sankara Sri Venkata, 121007 Faridabad (IN)
(74) Representative: Turner, Craig Robert

(57) **Abstract**

The present invention relates to an integrated method for methane rich biogas production from anaerobe systems using multiple reactors and various types of on-site selective enriched microbial consortium production. Biomass/organic waste is converted to biogas rich in methane content and with a minimum or absence of hydrogen suphide content at reduced hydraulic retention time (HRT). More particularly, the present invention relates to an on-site enrichment of selective microbial consortia with specified biochemical functions and their dosage into the respective digester at regular time intervals which improves the biomass conversion through biochemical steps, resulting into maximum production of methane rich biogas in a self-controlled biochemical function inside each bioreactor in comparatively lesser time intervals.

## Description

### FIELD OF THE INVENTION

The present invention relates to the field of biogas production. More particularly, the invention relates to a method for production of methane rich biogas in an anaerobic system. The invention also relates to an on-site enrichment of selective microbe with specified biochemical function, making the process more time and cost effective.

### BACKGROUND OF THE INVENTION

Anaerobic digestion is one of the most prominent technologies being considered in the realm of waste to energy sector. Various technologies have been developed for anaerobic digestion of organic waste and number of plants have been set up across the globe, but several issues still need to be addressed to make this technology commercially profitable and its implementation in big sized plants. Anaerobic digestion of biodegradable waste includes important metabolic steps, viz., hydrolysis, acidogenesis, acetogenesis, and methanogenesis, involving different metabolic reactions carried by synergistic interaction among various participating microbial communities. The existing technologies use agitation in either large single reactor or two bioreactors operating in parallel mode. Cross reaction of the metabolic reactions, short channelling of the partly digested feedstock, fluctuations in redox balance and pH inside the digester are some major challenges, which need to be addressed.

WO 2005/000748 discloses a biogas producing facility wherein organic waste is subjected to a digesting step in a first reactor, and subsequently to an anaerobic hydrolysis step in an anaerobic tank, whereby the energy content of material that was not digested in the first reactor is made available for bacterial digestion and thus, the hydrolysed material is fed into a second reactor, or is fed back into the first reactor for further bacterial conversion into biogas. The anaerobic hydrolysis step significantly increases the produced amount of biogas compared to a similar facility without the hydrolysis step. This is believed to be caused by the fact that performing a hydrolysis process on a biomass with a high content of volatile and easily digestible and reactive volatiles induces a tendency for constituents of organic matter to denature or condense during hydrolysis into derivatives of organic matter that cannot be digested in the reactor.

US7799546B2 discloses a three-step bio methanation process to convert starch or sugary agricultural feed stock into a methane rich gas mixture in a cost-effective manner. The process brings about conversion of starch-rich or sugar-rich biomass into methane through three stages, namely, hydrolysis, acidogenesis and methane formation. The invention deploys enzymatic hydrolysis of agriculture residues in the first stage followed by acidogenesis and methanogenesis. However, said invention does not address the problem of pH fluctuations, adjusting with biochemical functions, withstanding the feedback inhibitions, digester without any moving part inside, minimizing the scum formation without any chemicals, etc.

US4781836A discloses a bio methanation process that produces organic acids in first stage followed by their separation using membrane technology, desorption of these acids and conversion of them into biomethane. This invention does not teach about the controlling the pH fluctuations, adjusting with biochemical functions, withstanding the feedback inhibitions, digester without any moving part inside, minimizing the scum formation without any chemicals, etc.

EP0302968A1 discloses a starter culture for the bio methanation of lactose containing waste, such as whey. This invention does not talk about bio methanation of solid organic waste and does not address the major challenges of the existing systems like, the controlling the pH fluctuations, adjusting with the biochemical functions, withstanding the feedback inhibitions, digester without any moving part inside, minimizing the scum formation without any chemicals, etc.

CN104651412A discloses a method subjecting feedstock to the CO₂ and H₂ and methanogens to produce methane gas. Further, industrial tail gas also been tested for methane production. However, this invention does not talk about converting waste organics into biogas and typical bio methanation process. Also, this invention does not address any of the existing challenges of bio methanation like controlling of pH fluctuations, adjusting with biochemical functions, withstanding the feedback inhibitions, digester without any moving part inside, minimizing the scum formation without any chemicals, etc.

US20160369303A1 discloses means and methods for the bio methanation using H₂ and CO₂ as feedstock. However, this invention does not talk about converting waste organics into biogas and typical bio methanation process. Also, this invention does not address any of the existing challenges of bio methanation like controlling of pH fluctuations, adjusting with biochemical functions, withstanding the feedback inhibitions, digester without any moving part inside, minimizing the scum formation without any chemicals, etc.

US9284203B2 discloses a process of producing the syngas from the wastewater sludge and converting the same to syngas to enrich the methane content. However, this invention does not talk about converting waste organics to biogas and typical bio methanation process. Also, this invention does not address any of the existing challenges of bio methanation like controlling of pH fluctuations, adjusting with biochemical functions, withstanding the feedback inhibitions, digester without any moving part inside, minimizing the scum formation without any chemicals, etc.

EP20189098.5 (Indian Patent Application 201921037470) discloses an inoculum of microbial consortium containing live 10 microorganisms. More particularly, it relates to a novel enviro-tolerant methane-producing microbial consortium and a method for the production of biogas having high methane content from organic wastes and biomass slurries. The microbial consortium disclosed production of a stable biogas without any seasonal variation impact. The microbial consortium comprises of acetolactic methanogens such as *Desulfovibrio* sp. (IOC-2), *Brevibacterium* sp. (IOC-5), *Methanothermobacter* sp. (IOC-12), *Methanolobus* sp. (IOC-6), *Thermotoga* sp. (IOC-8); hydrogenotrophic methanogens such as *Methanosarcina* sp. (IOC-1), *Clostridium* sp. (IOC-3), *Methanobacterium* sp. (IOC-4) and *Lactobacillus* sp. (IOC-11); methanotrophic archaea such as *Methanosaeta* sp. (IOC-7), *Moorella* sp. (IOC-10) and *Lactobacillus* sp. (IOC-11) and electroactive bacteria such as *Clostridium* sp. (IOC-3), *Methanosaeta* sp. (IOC-7), *Pyrococcus* sp. (IOC-7) and *Shewanella* sp. MTCC 25020.

WO2014009575A1 discloses a process of bio methanation under micro-aerophilic conditions that produce lower concentrations of H₂S. However, this invention does not talk about the impact of air ingression on biogas yield and methane content. Also, this invention does not address any of the existing challenges of bio methanation like controlling of pH fluctuations, adjusting with biochemical functions, withstanding the feedback inhibitions, digester without any moving part inside, minimizing the scum formation without any chemicals, etc.

Despite of the advancements in the field of biogas production, there remains problems which have not been addressed till date such as cross reaction of the metabolic reactions, short channelling of the partly digested feedstock, fluctuations in redox balance and pH inside the digester, etc. A brief list of challenges or problems known in the prior arts are listed below:
- Inefficient production of biogas due to incomplete digestion of organic waste;
- Short channelling of unprocessed organic waste from reactor;
- Low microbial activity due to dilution effect by addition of new feedstock on daily basis;
- Alteration of biochemical functions inside digester because of incoming inconsistent nature of feed;
- Aging of culture over time due to change in microbial dynamics;
- Long hydraulic and solid retention time;
- Low biogas yield with less methane content;
- Scum formation inside the digester; and
- pH fluctuation in the reactor.

### SUMMARY OF THE INVENTION

While the methods of bio methanation or anaerobic digestion known in the art talks about only conversion of organics to biogas, the present invention focuses on making the process both cost and time efficient by minimizing the pH fluctuations and reducing the hydraulic retention time.

The present invention relates to a sequential process for maximum biogas production with increased substrate utilization and reduced hydraulic retention time. In an alternative embodiment the present invention relates to an integrated method for methane rich biogas production from anaerobe systems using multiple reactors and various types of on-site selective enriched microbial consortium production. Biomass/organic waste is converted to biogas rich in methane content with minimum or no hydrogen sulphide content in a reduced hydraulic retention time (HRT). The on-site enrichment of selective microbe with specified biochemical function and its dosage into the respective digester at regular time intervals confines the biomass conversion to a particular biochemical step (hydrolysis or acidogenesis or acetogenesis or methanogenesis), which results in a self-controlled biochemical function inside each bioreactor leading to the possibility of maximum extraction of biogas in less time intervals. The dosing of onsite enriched hydrogen producing consortia facilitates enhanced hydrogenotrophic methanogenesis reaction (CO₂+H₂ → CH4) and sulphide oxidizing bacteria facilitates the oxidation of sulphide, thus producing methane rich and low sulphide biogas that significantly reduce the purification efforts.

One of the aspects of the present invention relates to a process for methane rich biogas production from organic wastes using multiple reactors with self-controlled biochemical functions through on-site selective enrichment of microbial consortium, said process comprising steps of
**(a) Preparing a feed:** Preparing an organic waste feed by subjecting the organic waste to a size reduction of 1-5 mm, through a process selected among cutting, shredding, and pulverization;
**(b) Pretreating the organic waste feed:** optionally pretreating the organic waste feed of reduced size through process selected among hydrothermal treatment, extrusion, ozonation, hydrodynamic cavitation and combination thereof;
**(c) Preparing a feed slurry:** mixing the organic waste feed obtained from step a) i with water to prepare a feed slurry having 25-50% of total solid (TS) (w/w);
**(d) Processing the feed slurry:** transferring the feed slurry obtained from step c) through a series of reactors, wherein each reactor is attached with a small on-site selective bioinoculant generation reactor, and wherein the bioinoculant generation reactor is of 1/500^{th} size of the biogas production bioreactor; and
**(e) Removal of H₂S and CO₂**: *In situ* removal of H₂S and CO₂ from final biogas obtained from step (d).

In one of the aspects of the present invention, the organic waste feed obtained in step a), is subjected to a pretreatment process, when the lignin content in the feed is more than 10% of the total organic waste and wherein the pretreatment process is selected among hydrothermal treatment, extrusion, ozonation, hydrodynamic cavitation and combination thereof.

In another aspect of the present invention the process describes that the reactors R1, R2, R4, R6, and R8 are connected in series for processing the feed slurry, and the reactors R2, R4, R6, and R8 are connected to reactors R3, R5, R7, and R9, respectively, for providing blend of microbes.

In still another aspect of the present invention, the process describes that the feed slurry used in reactors R2, R4, R6, and R8 is adjusted to a total solid (TS) percent of 8-12% (w/w) by addition of water.

In another aspect, the present invention describes that 80% of content of the reactors R3, R5, R7, and R9 is transferred to R2, R4, R6, and R8, respectively, at an interval 24 hours, and wherein the volume of R3, R5, R7, and R9 is restored using the combination of slurry from R2, R4, R6, and R8, respectively, and nutrient rich waste/wastewater like molasses, spent wash, fruit waste. Once the feed slurry is added in reactor R1, the reactor R1 is maintained at 70 - 90°C with intermittent mixing for 5-24 hours.

In another aspect the present invention describes the process wherein feed slurry in reactors R2, R4, and R6, is maintained at 30 - 50°C with intermittent mixing for 48 - 72 hours.

In another aspect the present invention describes the process wherein the feed slurry when transferred to reactor R8, is supplied along with FeCl₃ in 0.01-0.05% and the reactor R8 is maintained at 30 - 50°C with intermittent mixing for 72 - 144 hours.

In another aspect the present invention describes the process wherein the biogas rich in methane and reduced concentration of H₂S is collected from reactors R6 and R8.

In another aspect of the present invention, the blend of microbes is grown such that microbes grown in reactors R3 and R5 are supplied to reactors R2 and R4, respectively in the initial concentration of 10⁴ - 10⁵ CFU/g of the feed slurry, and wherein the blend of microbes comprises of hydrolytic acidogenic, acetogenic and methanogenic microbes.

In another aspect the present invention describes a blend of microbes grown in reactors R7 and R9 are supplied to reactors R6 and R8, respectively in the initial concentration of 10⁶ to 10¹² CFU/g of the feed slurry, and wherein the blend of microbes comprises of hydrolytic acidogenic, acetogenic, methanogenic, and sulfide oxidizing microbes.

In another aspect the present invention describes the process wherein the reactors R6 and R8 are additionally supplied with a blend of microbes comprising of hydrogen producing microbes from reactor R10, and wherein the ratio of the blend of microbes supplied (from R7 and R10 to R6) and (from R9 and R10 to R8) is in a ratio of 2:1, and wherein the R10 is maintained at 60 - 70 °C and the volume of R10 being restored by a combination of feed slurry from R8 and nutrient rich waste/wastewater like molasses, spent wash, fruit waste.

In another aspect, the present invention describes a process wherein the blend of microbes used, the blend comprises hydrolytic acidogenic, acetogenic and methanogenic microbes is selected from the group consisting of *Desulfovibrio sp.* (MTCC No. 25301), *Brevibacterium sp.* (MTCC 25254), *Methanothermobacter sp.* (MTCC No. 25268), *Methanolobus sp.* (MTCC No. 25302), *Thermotoga sp.* (MTCC No. 25304), *Methanosarcina sp.* (MTCC No. 25300), *Clostridium sp.* (MTCC No. 25264), *Methanobacterium sp.* (MTCC No. 25266) and *Lactobacillus sp.* (MTCC No. 25282), *Methanosaeta sp.* (MTCC No. 25303), *Moorella sp.* (MTCC No. 25267) and *Pyrococcus sp.* (MTCC No. 25305) and *Shewanella sp.* MTCC 25020.

In another aspect, the present invention describes a process wherein the blend of microbes comprises of hydrolytic acidogenic, acetogenic and methanogenic microbes is selected from the group consisting of *Desulfovibrio sp.* (MTCC No. 25301), *Brevibacterium sp.* (MTCC 25254), *Methanothermobacter sp.* (MTCC No. 25268), *Methanolobus sp.* (MTCC No. 25302), *Thermotoga sp.* (MTCC No. 25304), *Methanosarcina sp.* (MTCC No. 25300), *Clostridium sp.* (MTCC No. 25264), *Methanobacterium sp.* (MTCC No. 25266) and *Lactobacillus sp.* (MTCC No. 25282), *Moorella sp.* (MTCC No. 25267) and *Methanosaeta sp.* (MTCC No. 25303), *Pyrococcus sp.* (MTCC No. 25305) and *Shewanella sp.* (MTCC 25020) and wherein the sulfide oxidizing microbes are selected from the group consisting of *Pseudomonas stutzeri* (MTCC 25027), *Arthobacter sp.* (MTCC 25028), *Achromobacter xylooxidan* (MTCC 25024), *Bacillus subtilis* (MTCC 25026), *Pseudomonas aeruginosa* (MTCC 5389), *Lysinibacillus sp.* (MTCC 5666), *Pseudomonas putida* (MTCC 5385).

In another aspect, the present invention describes a process wherein the hydrogen producing microbe is *Enterobacter aerogenes MTCC 25016.*

In another aspect, the present invention describes a system for production of methane rich biogas from organic wastes comprising of:
a) series of reactors for processing of feed slurry; and
b) series of loop reactors connected in loop to reactors for processing of feed slurry and providing blend of microbes,
wherein the loop reactors are of 1/500^{th} volume of corresponding reactors for processing of feed slurry, and wherein the reactors for processing of feed slurry are provided with agitators and drumbeat sticks.

In still another aspect, the present invention describes a system for production of methane rich biogas from organic wastes, wherein the series of reactors for processing of feed slurry comprises of reactors R1, R2, R4, R6, and R8 connected in series, and wherein the series of loop reactors comprises of reactors R3, R5, R7, and R9 connected in loop to the reactors R2, R4, R6, and R8, respectively, and an additional loop reactor R10 connected to R6 and R8.

In still another aspect, the present invention describes a system for production of methane rich biogas from organic wastes, wherein the reactor R8 is of ≥ 3 times volume than that of R2/ R4 / R6, and wherein reactor R1 is maintained at 70 - 90°C, reactors R2, R4, R6, and R8 are maintained at 30 - 50°C, and wherein reactor R8 is provided with a supply of FeCl₃. Furthermore, the reactors R3 and R5 described above, are provided with a blend of microbes comprises of hydrolytic acidogenic, acetogenic and methanogenic microbes, which are supplied to reactors R2 and R4, respectively, and wherein the reactors R7 and R9 are provided with a blend of microbes comprises of hydrolytic acidogenic, acetogenic, methanogenic, and sulfide oxidizing microbes, which are supplied to reactors R6 and R8, respectively, and wherein the reactor R10 is provided with a blend of microbes comprising of hydrogen producing microbes, which is supplied to reactors R6 and R8.

### DESCRIPTION OF THE ACCOMPANYING DRAWINGS

**Figure 1****:** Schematic representation of anaerobic digestion process for production of methane rich biogas.
**Figure 2****:** Variation in pH through a single cycle of bio methanation using paddy straw as feedstock.
**Figure 3****:** Variation in pH of different reactors of present invention on daily basis during continuous mode operation using paddy straw as feed stock.
**Figure 4****:** Variation in pH through a single cycle of bio methanation using MSW as feedstock.

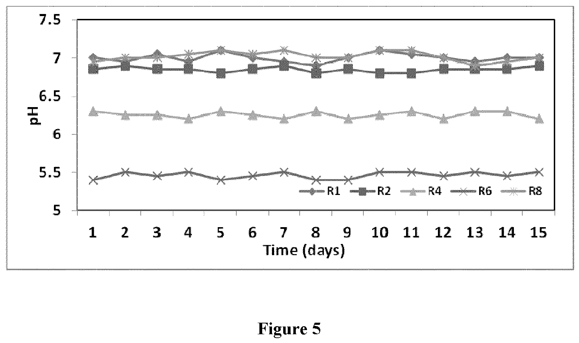

**Figure 5****:** Variation in pH of different reactors of present invention on daily basis during continuous mode production using paddy straw as feedstock.

### DETAILED DESCRIPTION OF THE INVENTION

Bio-methanization is a process by which organic material is microbiologically converted under anaerobic conditions to biogas. Bio-methanation of complex organic materials is performed by the interplay of essentially three microorganism groups, known as:
1. Hydrolytic fermentative bacteria;
2. Hydrogen producing acetogenic bacteria; and
3. Methanogenic bacteria which consume hydrogen and acetate.

In the process of bio-methanization the products of the first group are processed by the second microorganism group and the products of the second group are processed by the third group. As a result, the main products, methane, and carbon dioxide, are produced from complex biopolymers.

The present invention relates to a process of producing a biogas. More particularly, it relates to a sequential anaerobic digestion process for production of methane rich biogas using different kind of organic wastes. One main advantage of the present invention is that the method and a system for conversion of organic waste into biogas provides an increased yield of biogas along with the process being more time and cost effective.

Existing methods for bio methanation or anaerobic digestion talks about only the conversion of organics to biogas. However, the present invention focuses on minimizing the short channelling of unprocessed organic waste from reactor, self-control biochemical functions during the whole process, lowering down the pH fluctuations, maintaining high microbial count of log-phase/young consortium of selective nature, stabilizing different microbial population, lessening feedback inhibition, and minimizing the scum formation.

Further, the invention also relates to a novel method for production of methane rich biogas in anaerobic systems using multiple reactors and self-controlled biochemical functions through different types of on-site selective enriched microbial consortium production.

The present invention describes a sequential anaerobic digestion process for the production of methane rich biogas using different kind of organic wastes, wherein said process comprises of following steps:
**(a) Preparing a feed:** Preparing an organic waste feed by subjecting the organic waste to a size reduction of 1-5 mm, through a process selected among cutting, shredding, and pulverization;
**(b) Pretreating of organic waste feed:** optionally pretreating the organic waste feed of reduced size through process selected among hydrothermal treatment, extrusion, ozonation, hydrodynamic cavitation and combination thereof;
**(c) Preparing a feed slurry:** mixing the organic waste feed obtained from step a) i with water to prepare a feed slurry having 25-50% of total solid (TS) (w/w);
**(d) Processing the feed slurry:** transferring the feed slurry obtained from step c) through a series of reactors, wherein each reactor is attached with a small on-site selective bioinoculant generation reactor, and wherein the bioinoculant generation reactor is of 1/500^{th} size of the biogas production bioreactor; and
**(e) Removal of H₂S and CO₂**: *In situ* removal of H₂S and CO₂ from final biogas obtained from step (d).

The present invention describes the microbial consortium identified for added into series of reactors at different phases of the sequential process to produce methane rich biogas.

The present invention also describes the microbial blend identified for reactors R7 and R9 which include microbes for digesting the organic waste such as *Pseudomonas stutzeri* (MTCC 25027), *Arthobacter sp.* (MTCC 25028), *Achromobacter xylooxidan* (MTCC 25024), *Bacillus subtilis* (MTCC 25026), *Pseudomonas aeruginosa* (MTCC 5389), *Lysinibacillus sp.* (MTCC 5666), *Pseudomonas putida* (MTCC 5385).

The present invention also describes microbial blend identified to be used in the reactor R10 i.e., hydrogen producing culture, which consist of *Enterobacter aerogenes MTCC 25016.*

### EXAMPLES

### METHOD FOR PRODUCTION OF METHANE RICH BIOGAS

**Step 1. Size Reduction:** The biomass/organic waste is subjected for size reduction through cutting or shredding or pulverization and the size to be 1-5 mm size.

**Step 2. Pretreatment:** The size reduced biomass/organic waste is subjected to pretreatment if the lignin content is more than 10% of the biomass/organic waste. The pretreatment may include but not limited to hydrothermal treatment, extrusion, ozonation and hydrodynamic cavitation.

**Step 3. Slurry preparation:** The pre-treated biomass is mixed with water to make the feed slurry having 25-50% of TS (w/w) and send to R1. The water can be raw water or recycled water from the process. R1 is to be maintained at 70-90°C with intermittent mixing for 5-24 hrs.

**Step 4. Processing:** The feed slurry from the R1 is transferred to R2 along with addition of water to make the final TS in the reactor 8-12% (w/w). R2 is to be maintained at 30-50°C with intermittent mixing for 48-72 hours. R2 is connected in loop to R3 which is 1/500^{th} volume of R2. Microbial blend containing the microbes suitable for hydrolysis, acidogenesis, acetogenesis and methanogenesis are grown in R3 and added to R2. The initial microbial count of R2 is maintained to be 10⁴ to 10⁵ CFU/g of feed slurry. Intermittent mixing in R2 is provided by agitators or by circulating the slurry or by the gas produced. R2 have a set of drumbeat sticks on the exterior top of reactor to break the scum. R3 is in liquid communication at bottom of R2 and the microbes enriched in R3 are used to maintain microbial count inside R2. The microbe dosing from R3 to R2 is done by transferring 80% of R3 content to R2, at every 24 hours interval and the volume of R3 is make up using the combination of slurry from R2 and nutrient rich waste/wastewater like molasses, spent wash, fruit waste.

**Step 5.** Slurry from R2, after 48-72 hours, transferred to R4 which is connected in loop to R5. The R4 and R5 operation is done in exactly similar way to R2 and R3 operation with few modifications as follows. The feed slurry from the R2 is transferred to R4 and is maintained at 30-50°C with intermittent mixing for 48-72 hours. R4 is connected in loop to R5 which is 1/500^{th} volume of R4. Microbial blend containing the microbes suitable for hydrolysis, acidogenesis, acetogenesis and methanogenesis are grown in R5 and added to R4. The initial microbial count of R4 is maintained to be 10⁴ to 10⁵ CFU/g of feed slurry. Intermittent mixing in the R4 is provided by agitators or by circulating the slurry or by the gas produced. R4 have a set of drumbeat sticks on the exterior top of reactor to break the scum. R5 is in liquid communication at bottom of R4 and the microbes enriched in R5 are used to maintain microbial count inside R4. The microbe dosing from R5 to R4 is done by transferring 80% of R5 content to R4, at every 24h interval and the volume of R5 is make up using the combination of slurry from R4 and nutrient rich waste/wastewater like molasses, spent wash, fruit waste.

**Step 6.** Slurry from R4, after 48-72 hours is transferred to R6 which is connected in loop to R7. The R6 and R7 operation is done in exactly similar way to R4 and R5 operation with small modifications as follows. The feed slurry from the R4 is transferred to R6 and is maintained at 30-50°C with intermittent mixing for 48-72 hours. R6 is connected in loop to R7 which is 1/500^{th} volume of R6. Microbial blend containing the microbes suitable for hydrolysis, acidogenesis, acetogenesis and methanogenesis along with sulfide oxidizing bacteria are grown in R7 and added to R6. In addition, hydrogen producing consortia specifically grown in R10 is also added to R6. The ratio of cultures from R7 and R10 is maintained to be 2:1. The initial microbial count of R6 to be maintained 10⁶ to 10⁹ CFU/g of feed slurry. Intermittent mixing in the R6 is provided by agitators or by circulating the slurry. The biogas produced from R6 is collected to the storage balloon. R6 has a set of drumbeat sticks on the exterior top of reactor to break the scum. R7 is in liquid communication at bottom of R6 and the microbes enriched in R7 are used to maintain microbial count inside R6. The microbe dosing from R7 to R6 is done by transferring 80% of R7 content to R6, at every 24h interval and the volume of R7 is make up using the combination of slurry from R6 and nutrient rich waste/wastewater like molasses, spent wash, fruit waste.

**Step 7**. Slurry from R6, after 48-72 hours is transferred to R8 which is connected in loop to R9. The R8 and R9 operation is also done in more or less similar way to R6 and R7 operation but with modifications in size and operation as follows. The feed slurry from the R6 is transferred to R8 and is maintained at 30-50°C with intermittent mixing for 72-144 hours. While transferring the feed slurry from R6 to R8, the stream is mixed with FeCl₃ at 0.01-0.05% concentration. R8 is at least 3 times in volume to R2, R4 and R6 to manage the increased HRT. R8 is connected in loop to R9 which is 1/500^{th} volume of R8. Microbial blend containing the microbes suitable for hydrolysis acidogenesis, acetogenesis and methanogenesis along with sulfide oxidizing bacteria are grown in R9 and added to R8. In addition, hydrogen producing consortia specifically grown in R10 is also added to R8. The ratio of cultures from R9 and R10 is maintained to be 2:1. The initial microbial count of R8 to be maintained 10⁹ to 10¹² CFU/g of feed slurry. Intermittent mixing in the R8 is provided by agitators or by circulating the slurry. The biogas produced from R8 is collected to the storage balloon. R8 has a set of drumbeat sticks on the exterior top of reactor to break the scum. R9 is in liquid communication at bottom of R8 and the microbes enriched in R9 are used to maintain microbial count inside R8. The microbe dosing from R9 to R8 is done by transferring 80% of R9 content to R8, at every 24 hours interval and the volume of R9 is make up using the combination of slurry from R8 and nutrient rich waste/wastewater like molasses, spent wash, fruit waste.

**Step 8.** The hydrogen producing culture in R10 is grown continuously to dose the R7 and R9. The temperature for R10 is maintained at 60-70°C and the combination of feed slurry from R8 and nutrient rich waste/wastewater like molasses, spent wash, fruit waste.

**Step 9. Production of biogas:** The hydrogen rich biogas produced from R10 is also connected to R8 along with feed slurry to increase the methane content and reduce the CO₂ content of the biogas.

**Step 10.** Purification: Biogas produced from R6 and R8 transferred to balloon and is rich in methane due to hydrogenotrophic methanogenesis (CO₂+H₂→CH₄) facilitated by hydrogen producing culture and hydrogen rich biogas producing from R10. Further, the biogas is devoid of or having minimum concentration of H₂S due to the sulfide oxidizing bacteria. The biogas after subjecting to minimum purification can be compressed and filled in cylinder bank.

The seed bioinoculant used in the current invention is similar for R2 and R4, R6, R8 and R10. However, the onsite enrichment over the time changes the actual composition of the microbial blend and will sustain the composition due to regular dosing of the same.

The microbial blend used for R2 and R4 during seed include the microbial blend (i) acetolactic methanogens is selected from a group consisting of *Desulfovibrio sp.* (MTCC No. 25301), *Brevibacterium sp.* (MTCC 25254), *Methanothermobacter sp.* (MTCC No. 25268), *Methanolobus sp.* (MTCC No. 25302), *Thermotoga sp.* (MTCC No. 25304); (ii) hydrogenotrophic methanogens selected from the group consisting of *Methanosarcina sp.* (MTCC No. 25300), *Clostridium sp.* (MTCC No. 25264), *Methanobacterium sp.* (MTCC No. 25266) and *Lactobacillus sp.* (MTCC No. 25282); (iii) methanotrophic archaea selected from the group consisting *of Methanosaeta sp.* (MTCC No. 25303), *Moorella sp.* (MTCC No. 25267) and *Lactobacillus sp.* (MTCC No. 25282); and (iv) electroactive bacteria selected from a group consisting of *Clostridium sp.* (MTCC 25264), *Methanosaeta sp.* (MTCC No. 25303), *Pyrococcus sp.* (MTCC No. 25305) and *Shewanella sp.* MTCC 25020 (as disclosed in the Patent Application 201921037470).

Similarly, the seed bioinoculant used in R7 and R9 include all the microbes mentioned for R2 and R4 along with sulfide oxidizing bacteria that include but not limited to *Pseudomonas stutzeri* (MTCC 25027), *Arthobacter sp.* (MTCC 25028), *Achromobacter xylooxidan* (MTCC 25024), *Bacillus subtilis* (MTCC 25026), *Pseudomonas aeruginosa* (MTCC 5389), *Lysinibacillus sp.* (MTCC 5666), *Pseudomonas putida* (MTCC 5385).

The seed bioinoculant used in R10 is a microbial blend of following but not limited to *Enterobacter aerogenes MTCC 25016.*

### EXAMPLE 1:

### BIO METHANATION OF DIFFERENT TYPES OF ORGANIC WASTE

The efficiency of bio methanation process was evaluated using different feedstock, viz., organic fraction of municipal solid waste (MSW), press mud, cattle dung, paddy straw and water hyacinth (Table 1). Different combinations of experiments were performed with the selected feedstock including the pretreatment options. All the selected feedstock checked and removed any non-biodegradable materials. Shredding/size reduction is carried out for MSW, paddy straw and water hyacinth but for press mud and cattle dung size reduction is not required. Different pretreatment options included for MSW, paddy straw and water hyacinth but no pretreatment is required for press mud and cattle dung. TS content for R1 in case of MSW, press mud, cattle dung and water hyacinth kept as such without any dilution but in case of paddy straw, the TS content of R1 maintained at 50%. The hydraulic retention time is different for each feedstock based on their complexity as designated in Table 1. Typical single stage bio methanation using the same biocatalyst is considered as control operation. After the designated pretreatment, feedstock is subjected to bio methanation. Temperature of the digester is maintained at 50°C and % TS is maintained like R2 digester of present invention.

**Table 1: Experimental conditions adapted to evaluate the potential of present invention using different feedstocks**

| **Exp No.** | **Feedstock** | **%TS** | **Size reduction (mm)** | **Pretreatme nt adapted** | **Condition s for R1 (%TS loading, Temp. (⁰C) and HRT (h))** | **Condition s for R2 (%TS loading, Temp. (⁰C) and HRT (h))** | **Conditions for R4 and R6 (%TS loading, Temp. (⁰C) and HRT (h))** | **Conditions for R8 (%TS loading, Temp. (⁰C) and HRT (h), FeCl3 (%))** |
|---|---|---|---|---|---|---|---|---|
| 1 | Municipal Solid Waste (MSW) | 25-35 | Crushing (1-2 mm) | Hydro-thermal (121 °C and 15 lbs. for 15 min) | %TS, 25 Temp., 60 HRT, 12 | %TS, 10 Temp., 30 HRT, 48 | %TS, <10 Temp., 30 HRT, 48 | %TS, <10 Temp., 30 HRT, 72 FeC13, 0.02 |
| 2 | | | | Ozonation (dose at 5 g/h for 30 min) | %TS, 30 Temp., 50 HRT, 12 | %TS, 12 Temp., 40 HRT, 60 | %TS, <12 Temp., 40 HRT, 60 | %TS, <12 Temp., 40 HRT, 84 FeC13, 0.01 |
| 3 | | | | Extrusion (50 kg/h) | %TS, 35 Temp., 70 HRT, 8 | %TS, 12 Temp., 40 HRT, 72 | %TS, <12 Temp., 40 HRT, 72 | %TS, <12 Temp., 40 HRT, 96 FeC13, 0.02 |
| 4 | Press Mud | 25-30 | NR | NR | %TS, 26 Temp., 50 HRT, 8 | %TS, 13 Temp., 40 HRT, 48 | %TS, <13 Temp., 40 HRT, 48 | %TS, <13 Temp., 40 HRT, 96 FeC13, 0.04 |
| 5 | Cattle dung | 18-26 | NR | NR | %TS, 21 Temp., 70 | %TS, 10 Temp., 40 | %TS, <10 Temp., 40 | %TS, <10 Temp., 40 |
| | | | | | HRT, 5 | HRT, 48 | HRT, 48 | HRT, 72 FeC13, 0.05 |
| 6 | Paddy straw | 85-90 | Shredding (0.5 mm) | Hydro-thermal (121 °C and 15 lbs. for 15 min) | %TS, 40 Temp., 80 HRT, 18 | %TS, 10 Temp., 40 HRT, 48 | %TS, <10 Temp., 40 HRT, 72 | %TS, <10 Temp., 40 HRT, 120 FeC13, 0.02 |
| 7 | | | Shredding (3 mm) | Hydrodyna mic cavitation | %TS, 50 Temp., 90 HRT, 12 | %TS, 12 Temp., 50 HRT, 72 | %TS, <12 Temp., 50 HRT, 72 | %TS, <12 Temp., 50 HRT, 144 FeC13, 0.01 |
| 8 | | | Shredding (5 mm) | Extrusion (50 kg/h) | %TS, 40 Temp., 85 HRT, 24 | %TS, 12 Temp., 45 HRT, 60 | %TS, <12 Temp., 45 HRT, 60 | %TS, <12 Temp., 45 HRT, 132 FeC13, 0.01 |
| 9 | Water Hyacinth | 15-25 | Shredding (1 mm) | Hydrodyna mic cavitation | %TS, 25 Temp., 60 HRT, 15 | %TS, 12 Temp., 40 HRT, 48 | %TS, <12 Temp., 40 HRT, 72 | %TS, <12 Temp., 40 HRT, 120 FeC13, 0.02 |
| 10 | | | Shredding (2 mm) | Ozonation (dose at 10 g/h for 30 min) | %TS, 25 Temp., 75 HRT, 10 | %TS, 12 Temp., 45 HRT, 60 | %TS, <12 Temp., 45 HRT, 60 | %TS, <12 Temp., 45 HRT, 132 FeC13, 0.03 |
| 11 | | | Shredding (4 mm) | Extrusion (50 kg/h) | %TS, 25 Temp., 80 HRT, 14 | %TS, 12 Temp., 50 HRT, 72 | %TS, <12 Temp., 50 HRT, 72 | %TS, <12 Temp., 50 HRT, 144 FeC13, 0.02 |

**Table 2: Comparative evaluation of present invention over the conventional single stage bio methanation**

| **Exp No.** | **%TS removal** | | **% COD removal** | | **Biogas yield (m3/Ton)** | | **Methane Content (%)** | | **H2S content (ppm)** | | **HRT (hrs.)** | |
|---|---|---|---|---|---|---|---|---|---|---|---|---|
| | **Control (Single stage)** | **P.I** | **Control (Single stage)** | **P.I.** | **Control (Single stage)** | **P.I.** | **Control (Single stage)** | **P.I.** | **Control (Single stage)** | **P.I.** | **Control (Single stage)** | **P.I.** |
| 1 | 48-51 | 74-77 | 52-56 | 76-79 | 60-75 | 110-128 | 69-70 | 72-74 | 900-1700 | 40-80 | 336-442 | 228-280 |
| 2 | 52-56 | 78-82 | 57-63 | 75-78 | 64-78 | 116-140 | 68-70 | 74-76 | 900-1700 | 40-80 | 336-442 | 228-280 |
| 3 | 50-54 | 75-79 | 54-61 | 74-77 | 62-72 | 112-134 | 68-71 | 73-76 | 900-1700 | 40-80 | 336-442 | 228-280 |
| 4 | 58-61 | 76-83 | 63-65 | 79-87 | 80-90 | 144-162 | 69-71 | 72-76 | 800-1600 | 60-90 | 336-442 | 240-250 |
| 5 | 62-66 | 73-75 | 66-70 | 78-81 | 45-60 | 105-124 | 70-72 | 71-76 | 1400-2700 | 70-110 | 336-442 | 220-230 |
| 6 | 40-46 | 63-67 | 46-53 | 69-76 | 242-266 | 324-356 | 68-70 | 72-75 | 400-1000 | 20-50 | 504-576 | 330-380 |
| 7 | 51-54 | 72-75 | 55-59 | 76-81 | 256-288 | 328-359 | 69-71 | 74-75 | 400-1000 | 20-50 | 504-576 | 330-380 |
| 8 | 48-52 | 69-73 | 53-56 | 74-78 | 250-274 | 326-352 | 69-71 | 74-76 | 400-1000 | 20-50 | 504-576 | 330-380 |
| 9 | 50-56 | 76-81 | 54-61 | 80-87 | 62-71 | 82-93 | 67-70 | 73-76 | 500-900 | 30-70 | 504-576 | 300-370 |
| 10 | 52-57 | 78-84 | 55-61 | 82-86 | 66-78 | 86-95 | 68-71 | 71-74 | 500-900 | 30-70 | 504-576 | 300-370 |
| 11 | 49-53 | 78-82 | 54-57 | 81-86 | 64-72 | 84-89 | 69-72 | 72-75 | 500-900 | 30-70 | 504-576 | 300-370 |

| | | | | | | | | | | | | |
|---|---|---|---|---|---|---|---|---|---|---|---|---|
| P.I. in the above table stands for present invention. | | | | | | | | | | | | |

### EXAMPLE 2:

### MONITORING OF pH FLUCTUATION

Experiments were performed using MSW and paddy straw in continuous mode for establishing the pH stability of present invention against the conventional bio methanation. The selected feedstock was checked, and all the non-biodegradable material was removed from it manually. Shredding or size reduction is carried out followed by designated pretreatment. TS content for R1 in case of MSW is kept as such without any dilution but in case of paddy straw, the TS content of R1 maintained at 50%. After the designated pretreatment, feedstock is subjected to bio methanation. Temperature of the digester is maintained at 50°C and % TS is maintained like R2 in the digester. The hydraulic retention time adapted in each reactor is different for each feedstock based on their complexity and the same is designated in Table 3. Typical single stage bio methanation using the same biocatalyst is considered as control operation for both the feedstocks. HRT for control operation considered as 14 days for MSW and 24 days for paddy straw. Rest all other conditions maintained similar to test conditions.

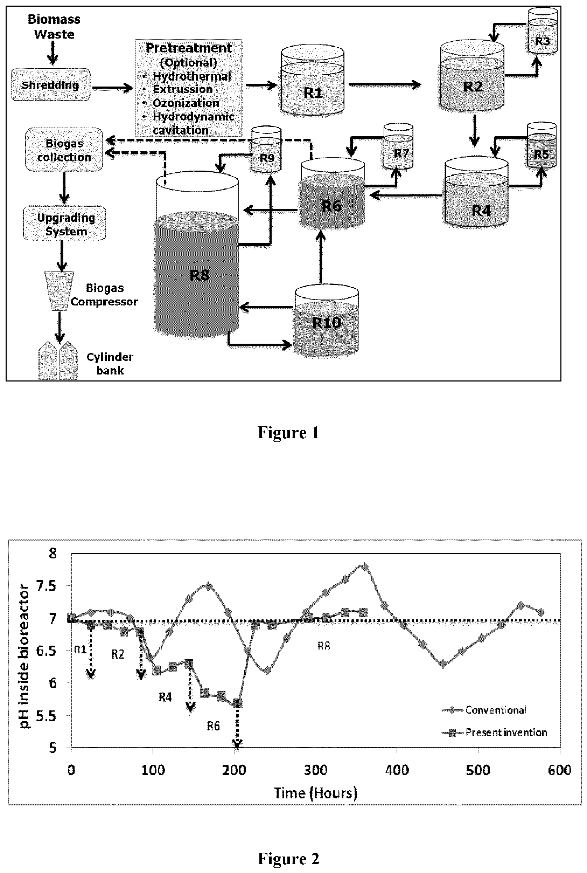

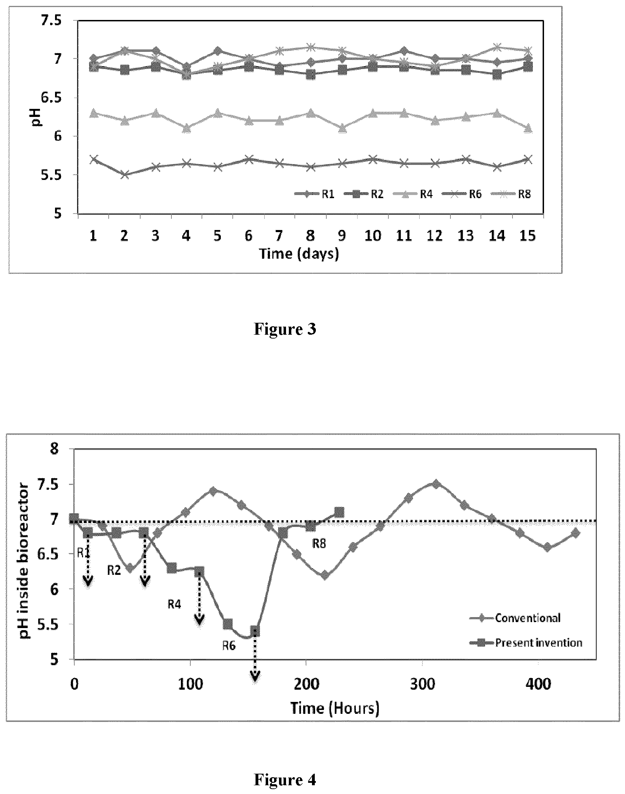

The results obtained from the experiment with paddy straw and MSW has been shown in figures 1 and 3 wherein a clear demarcation of pH stability against the conventional bio methanation is observed. The operating pH of each reactor has showed mere fluctuation for number of days using both MSW and paddy straw as feedstock (Figure 2 and 4). This behavior in each reactor is due to self-controlled biochemical function of each reactor which helps in the proliferation of stable microbial dynamics in turn resulting in higher biogas yields, high substrate conversion and low HRT.

**Table 3: Experimental conditions adapted for establishment of pH stability**

| **Feedstock** | **Pretreatment adapted and other conditions** | **Conditions for R1 (%TS loading, Temp. (⁰C) and HRT (h))** | **Conditions for R2 (%TS loading, Temp. (⁰C) and HRT (h))** | **Conditions for R4 and R6 (%TS loading, Temp. (⁰C) and HRT (h))** | **Conditions for R8 (%TS loading, Temp. (⁰C) and HRT (h), FeCl3 (%))** |
|---|---|---|---|---|---|
| MSW (%TS: 25-35) | Crushing (1-2 mm) Hydro-thermal (121 °C and 15 lbs. for 15 min) | %TS, 25 Temp., 60 HRT, 12 | %TS, 10 Temp., 30 HRT, 48 | %TS, <10 Temp., 30 HRT, 48 | %TS, <10 Temp., 30 HRT, 72 FeC13, 0.02 |
| Paddy straw (%TS: 85-90) | Shredding (5 mm) | %TS, 40 Temp., 85 HRT, 24 | %TS, 12 Temp., 45 HRT, 60 | %TS, <12 Temp., 45 HRT, 60 | %TS, <12 Temp., 45 HRT, 132 FeC13, 0.01 |
| | Extrusion (50 kg/h) | | | | |

Technical advancements contributed by the present invention:
- Minimizing the short channelling of unprocessed organic waste from reactor
- Self-controlled biochemical functions during whole process
- High biogas yield with enriched methane and low/nil sulphide content
- Maintaining high microbial count of log-phase/young consortium of selective nature
- Low pH fluctuation inside the reactor
- Less product feedback inhibition
- Shorter hydraulic and solid retention time
- Stabilization of different microbial populations
- No moving part inside the reactor
- Minimizing the scum formation

## Claims

1. A process for methane rich biogas production from organic wastes using multiple reactors with self-controlled biochemical functions through on-site selective enrichment of microbial consortium, said process comprising steps of
a) **Preparing a feed:** Preparing an organic waste feed by subjecting the organic waste to a size reduction of 1-5 mm, through a process selected among cutting, shredding, and pulverization;
b) **Pretreating the organic waste feed:** optionally pretreating the organic waste feed of reduced size through process selected among hydrothermal treatment, extrusion, ozonation, hydrodynamic cavitation and combination thereof;
c) **Preparing a feed slurry:** mixing the organic waste feed with water to prepare a feed slurry having 25-50% of total solid (TS) (w/w);
d) **Processing the feed slurry:** transferring the feed slurry obtained from step c) through a series of reactors, wherein each reactor is attached with a small on-site selective bioinoculant generation reactor, and wherein the bioinoculant generation reactor is of 1/500^{th} size of the biogas production bioreactor; and
e) **Removal of H₂S and CO₂**: *In situ* removal of H₂S and CO₂ from final biogas obtained from step (d).

2. The process, as claimed in claim 1, wherein the organic waste feed obtained in step a) is subjected to a pretreatment process, when the lignin content is more than 10% of the total organic waste.

3. The process as claimed in claim 1, wherein the series of reactors comprises of reactors R1, R2, R4, R6, and R8 connected in series for processing the feed slurry, wherein the feed slurry in reactors R2, R4, R6, and R8 is adjusted to a total solid (TS) percent of 8-12% (w/w) by addition of water, and wherein the reactors R2, R4, R6, and R8 are connected to reactors R3, R5, R7, and R9, respectively, for providing blend of microbes.

4. The process as claimed in claim 3, wherein 80% of content of the reactors R3, R5, R7, and R9 is transferred to R2, R4, R6, and R8, respectively, at an interval 24 hours, and wherein the volume of R3, R5, R7, and R9 is restored using the combination of slurry from R2, R4, R6, and R8, respectively, and nutrient rich waste/wastewater like molasses, spent wash, fruit waste.

5. The process, as claimed in claim 3, wherein the feed slurry in reactor R1 is maintained at 70 - 90°C with intermittent mixing for 5 - 24 hours, wherein the feed slurry in reactors R2, R4, and R6, is maintained at 30 - 50°C with intermittent mixing for 48 - 72 hours.

6. The process as claimed in claim 3, wherein the feed slurry in reactor R8 is additionally supplied with FeCl₃ in 0.01-0.05% and the reactor R8 is maintained at 30 - 50°C with intermittent mixing for 72 - 144 hours, and wherein the biogas rich in methane and reduced concentration of H₂S is collected from reactors R6 and R8.

7. The process as claimed in claim 3, wherein the blend of microbes grown in reactors R3 and R5 are supplied to reactors R2 and R4, respectively in the initial concentration of 10⁴ - 10⁵ CFU/g of the feed slurry, and wherein the blend of microbes comprises of hydrolytic acidogenic, acetogenic and methanogenic microbes.

8. The process as claimed in claim 3, wherein the blend of microbes grown in reactors R7 and R9 are supplied to reactors R6 and R8, respectively in the initial concentration of 10⁶ to 10¹² CFU/g of the feed slurry, and wherein the blend of microbes comprises of hydrolytic acidogenic, acetogenic, methanogenic, and sulfide oxidizing microbes.

9. The process as claimed in claim 3, wherein the reactors R6 and R8 are additionally supplied with a blend of microbes comprising of hydrogen producing microbes from reactor R10, and wherein the ratio of the blend of microbes supplied (from R7 and R10 to R6) and (from R9 and R10 to R8) is in a ratio of 2:1, and wherein the R10 is maintained at 60 - 70 °C and the volume of R10 being restored by a combination of feed slurry from R8 and nutrient rich waste/wastewater like molasses, spent wash, fruit waste.

10. The process as claimed in claim 7, wherein the blend of microbes comprising hydrolytic acidogenic, acetogenic and methanogenic microbes is selected from the group consisting of *Desulfovibrio sp.* (MTCC No. 25301), *Brevibacterium sp.* (MTCC 25254), *Methanothermobacter sp.* (MTCC No. 25268), *Methanolobus sp.* (MTCC No. 25302), *Thermotoga sp.* (MTCC No. 25304), *Methanosarcina sp.* (MTCC No. 25300), *Clostridium sp.* (MTCC No. 25264), *Methanobacterium sp.* (MTCC No. 25266) and *Lactobacillus sp.* (MTCC No. 25282), , *Moorella sp.* (MTCC No. 25267) and, *Methanosaeta sp.* (MTCC No. 25303), *Pyrococcus sp.* (MTCC No. 25305) and *Shewanella sp.* (MTCC No. 25020).

11. The process as claimed in claim 8, wherein the blend of microbes comprising hydrolytic acidogenic, acetogenic and methanogenic microbes is selected from the group consisting of *Desulfovibrio sp.* (MTCC No. 25301), *Brevibacterium sp.* (MTCC 25254), *Methanothermobacter sp.* (MTCC No. 25268), *Methanolobus sp.* (MTCC No. 25302), *Thermotoga sp.* (MTCC No. 25304 ), *Methanosarcina sp.* (MTCC No. 25300), *Clostridium sp.* (MTCC No. 25264), *Methanobacterium sp.* (MTCC No. 25266) and *Lactobacillus sp.* (MTCC No. 25282), , *Moorella sp.* (MTCC No. 25267) and *Methanosaeta sp.* (MTCC No. 25303), *Pyrococcus sp.* (MTCC No. 25305) and *Shewanella sp.* MTCC 25020 and wherein the sulfide oxidizing microbes are selected from the group consisting of *Pseudomonas stutzeri* (MTCC 25027), *Arthobacter sp.* (MTCC 25028), *Achromobacter xylooxidan* (MTCC 25024), *Bacillus subtilis* (MTCC 25026), *Pseudomonas aeruginosa* (MTCC 5389), *Lysinibacillus sp.* (MTCC 5666), *Pseudomonas putida* (MTCC 5385).

12. The process as claimed in claim 9, wherein the hydrogen producing microbes is *Enterobacter aerogenes* (MTCC 25016).

13. A system for production of methane rich biogas from organic wastes comprising of
a) series of reactors for processing of feed slurry; and
b) series of loop reactors connected in loop to reactors for processing of feed slurry and providing blend of microbes,
wherein the series of reactors for processing of feed slurry comprises of reactors R1, R2, R4, R6, and R8 connected in series, and wherein the series of loop reactors comprises of reactors R3, R5, R7, and R9 connected in loop to the reactors R2, R4, R6, and R8, respectively, and an additional loop reactor R10 connected to R6 and R8, and
wherein the loop reactors are of 1/500^{th} volume of corresponding reactors for processing of feed slurry, and wherein the reactors for processing of feed slurry are provided with agitators and drumbeat sticks.

14. The system as claimed in claim 13, wherein the reactor R8 is of ≥ 3 times volume than that of R2/ R4 / R6, and wherein reactor R1 is maintained at 70 - 90°C, reactors R2, R4, R6, and R8 are maintained at 30 - 50°C, and wherein reactor R8 is provided with a supply of FeCl₃.

15. The system as claimed in claim 13, wherein the reactors R3 and R5 are provided with a blend of microbes comprises of hydrolytic acidogenic, acetogenic and methanogenic microbes, which are supplied to reactors R2 and R4, respectively, and wherein the reactors R7 and R9 are provided with a blend of microbes comprises of hydrolytic acidogenic, acetogenic, methanogenic, and sulfide oxidizing microbes, which are supplied to reactors R6 and R8, respectively, and wherein the reactor R10 is provided with a blend of microbes comprising of hydrogen producing microbes, which is supplied to reactors R6 and R8.
